# EUROPÄISCHE PATENTANMELDUNG

(11) **EP 1 810 964 A1**
(43) Veröffentlichungstag der Anmeldung: **25.07.2007**
(21) Anmeldenummer: 06001336.4
(22) Anmeldetag: 23.01.2006
(51) Int. Cl.: C07C 233/30, C07D 211/88

(54) **Verfahren zur Herstellung von Dicarbonsäureimiden**

(71) Anmelder: Siegfried Ltd., 4800 Zofingen (CH)
(72) Erfinder: Landmesser, Thomas, 8005 Zürich (CH); Bonnier, Hans, 4663 Aarburg (CH)
(74) Vertreter: Behnisch, Werner

(57) **Zusammenfassung**

Die vorliegende Erfindung betrifft ein Verfahren zur Herstellung eines Carbonsäureimids der allgemeinen Formel

R¹-(CO)-(NR³)-(CO)-R² (I),

wobei ein Carbonsäureanhydrid der allgemeinen Formel

R¹-(CO)-O-(CO)-R² (II)

mit Harnstoff oder einem Harnstoffderivat der Form (R³HN)-(CO)-(NR³H) in einem Lösemittel umgesetzt wird. Insbesondere kann das Verfahren zur Herstellung von Thalidomid eingesetzt werden.

## Beschreibung

Die vorliegende Erfindung betrifft ein Verfahren zur Herstellung von Dicarbonsäureimiden aus den entsprechenden Dicarbonsäureanhydriden und Harnstoff oder Harnstoffderivaten.

Dicarbonsäureimide kommen als Baustein in vielen pharmazeutisch genutzten Stoffen vor. Einer der bekanntesten Wirkstoffe mit einer Dicarbonsäureimidfunktion ist Thalidomid. Erstmalig wurde es im Jahr 1954 beschrieben. Anfänglich wurde Thalidomid als Sedativum verwendet. In den letzen Jahren zeigte sich jedoch, dass Thalidomid, wie auch dessen Derivate, bei der Behandlung verschiedener Krankheiten wie z.B. Lepra, rheumatoider Arthritis, AIDS, Morbus Crohn sowie bei Krebserkrankungen eingesetzt werden kann. Thalidomid wirkt immun-suppressiv und immun-modulierend.

Für die Synthese von Thalidomid sind in der Literatur verschiedene Wege bekannt. Eine Übersicht ist in "Axel Kleemann and Jürgen Engel, Pharmaceutical Substances, Thieme Verlag, Stuttgart, 4.Auflage" auf den Seiten 2005-2007 dargestellt. Die gängigste Variante geht von Phthalsäureanhydrid aus, das mit Glutaminsäure zu N-Phthaloyl-Glutaminsäure umgesetzt wird. Diese wird mit Essigsäureanhydrid zu N-Phthaloylglutaminsäureanhydrid umgesetzt. Das Anhydrid wiederum wird unter Einwirkung von Harnstoff in der Schmelze in Thalidomid überführt. Dabei treten die üblichen Probleme für Reaktionen mit Gasentwicklung in der Schmelze auf, wie z.B. übermäßige Schaumbildung oder schlechte Löslichkeit des Produktgemisches und somit erschwerte Aufbereitung des Produktgemisches.

Daher wäre es hilfreich ein Verfahren zu kennen, das die Synthese von Dicarbonsäureimiden, insbesondere von Thalidomid und seinen Derivaten, auf dem Wege der leichter zu kontrollierenden Reaktionsführung in Lösung ermöglicht. Das Ziel der vorliegenden Erfindung ist es, ein Verfahren zur Synthese von Dicarbonsäureimiden in Lösung bereitzustellen.

Diese Aufgabe wird durch das Verfahren des unabhängigen Anspruchs gelöst. Vorteilhafte Ausgestaltungen werden in den Unteransprüchen fortgeführt.

Überraschenderweise haben die Erfinder der vorliegenden Anmeldung herausgefunden, dass eine Umsetzung von Säureanhydriden mit Harnstoff in einem hochsiedenden Lösemittel zur Synthese von Dicarbonsäureimiden führt. Diese Reaktionsführung erlaubt somit z.B. die Synthese von Thalidomid ausgehend von N-Phthaloylglutaminsäureanhydrid. Beispielhaft ist die Synthese von Thalidomid ausgehend von N-Phthaloylglutaminsäureanhydrid in Schema 1 mit Sulfolan (Tetrahydrothiophen-1,1-dioxid) als Lösemittel dargestellt.

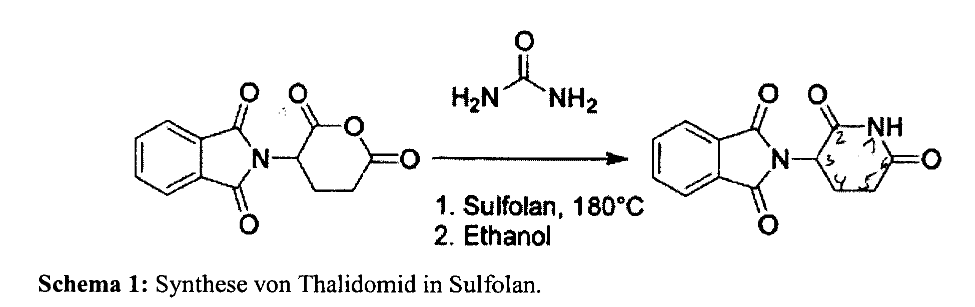

Die Erfindung stellt ein Verfahren zur Herstellung eines Dicarbonsäureimides der allgemeinen Formel R¹-(CO)-(NR³)-(CO)-R² (I) bereit, wobei ein Dicarbonsäureanhydrid der allgemeinen Formel R¹-(CO)-O-(CO)-R² (II) mit Harnstoff oder einem Harnstoffderivat der Form (R³HN)-(CO)-(NR³H) in einem Lösemittel zu dem Dicarbonsäureimid (I) umgesetzt, wobei R¹, R² und R³ unabhängig voneinander substituiertes oder unsubstituiertes, geradkettiges, verzweigtes oder cyclisches C₁-C₁₀-Alkyl, C₂-C₁₀-Alkenyl, C₂-C₁₀-Alkinyl, C₄-C₁₀-Aryl, C₄-C₁₀-Heteroaryl sein können, oder wobei R¹ und R² zur Bildung eines Ringes miteinander verbunden sein können, und/oder wobei R³ auch H sein kann. Wenn R¹ und R² zur Bildung eines Ringes miteinander verbunden sind, so bilden sie zusammen einen zweiwertigen Rest R⁴. Die Reste R¹ bis R⁴ können jeweils unsubstituiert, einfach oder auch mehrfach substituiert sein. Wesentlich für die Erfindung ist die Umsetzung des Dicarbonsäureanhydrides mit Harnstoff oder einem Derivat von Harnstoff zu einem entsprechenden Dicarbonsäureimid.

In einer bevorzugten Ausführungsform der Erfindung wird ein Herstellungsverfahren für Dicarbonsäureimide der allgemeinen Formel (III) 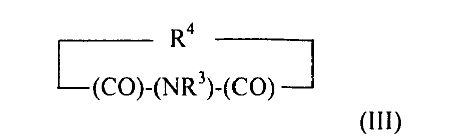 bereitgestellt, wobei R³ wie oben definiert ist, und R⁴ ein zweiwertiger Rest wie R¹ oder R² ist, also R⁴ ein substituiertes oder unsubstituiertes, geradkettiges, verzweigtes oder cyclisches C₁-C₁₀-Alkandiyl, C₂-C₁₀-Alkenylen, C₂-C₁₀-Alkinylen, C₄-C₁₀-Arylen, C₄-C₁₀-Heteroarylen sein kann. Bevorzugt wird das Verfahren eingesetzt, um substituierte oder unsubstituierte Piperidin-2,6-dione herzustellen, wobei R⁴ substituiertes oder unsubstituiertes 1,3-Propandiyl, besonders bevorzugt unsubstituiertes oder substituiertes 1-Phthalimido-1,3-propandiyl ist, und insbesondere unsubstituiertes 1-Phthalimido-1,3-propandiyl zur Synthese von Thalidomid.

Bei dem erfindungsgemäßen Verfahren werden hochsiedende Lösemittel oder Lösemittelgemische eingesetzt, bevorzugt Lösemittel mit einem Siedepunkt unter Normaldruck von über 150°C, noch bevorzugter von über 170°C, und am bevorzugtesten von über 190°C. Dabei können die Lösemittel ausgewählt werden aus aprotischen Sulfonen wie zum Beispiel Tetrahydrothiophen-1,1-dioxid (Sulfolan), gesättigten Lactamen wie zum Beispiel N-Methylpyrrolidon (NMP), Carbonsäureamiden wie zum Beispiel N,N-Dimethylacetamid (DMA) oder Formamid, Ethern wie zum Beispiel Diphenylether, Harnstoffen wie zum Beispiel 1,3-Dimethyl-2-imidazolidinon (DMI), Polyethylenglycolen wie zum Beispiel Diethylenglycol-diethylether, ein- oder mehrfach alkylsubstituierten Aromaten wie zum Beispiel Diethylbenzol, Pseudocumol, Cumol oder Mesitylen, ionischen Flüssigkeiten wie zu Beispiel 1-Ethyl-3-methyl-imidazoliumtosylat, Siloxanen wie zum Beispiel Decamethylcyclopentasiloxan, gesättigten oder teilweise gesättigten Carbocyclen wie zum Beispiel Tetralin oder Decalin, Kohlensäureestern wie zum Beispiel Propylencarbonat und aromatischen Aminen wie zum Beispiel N,N-Diethylanilin, oder deren Mischungen. Besonders bevorzugt ist hierbei Tetrahydrothiophen-1,1-dioxid (Sulfolan).

| *Gruppe* | *Produkte* |
|---|---|
| aprotische Sulfone | Tetrahydrothiophen-1,1-dioxid (Sulfolan) |
| gesättigte Lactame | N-Methylpyrrolidon (NMP) |
| Carbonsäureamide | N,N-Dimethylacetamid (DMA) Formamid |
| Ether | Diphenylether |
| Harnstoffe | 1,3-Dimethyl-2-imidazolidinon (DMI) |
| Polyethylenglycole | Diethylenglycol-diethylether |
| ein- oder mehrfach alkylsubstituierte Aromaten | Diethylbenzol Pseudocumol Cumol Mesitylen |
| ionische Flüssigkeiten | 1-Ethyl-3-methyl-imidazoliumtosylat |
| Siloxane | Decamethylcyclopentasiloxan |
| gesättigte oder teilweise gesättigte Carbocyclen | Decalin Tetralin |
| Kohlensäureester | Propylencarbonat |
| aromatische Amine | N,N-Diethylanilin |

Bevorzugt wird das Verfahren bei Normaldruck durchgerührt. Es ist jedoch auch möglich, das Verfahren bei Über- oder Unterdruck durchzuführen. Es ist auch möglich, die Umsetzung unter einer Schutzgasatmosphäre, wie beispielsweise Stickstoff oder Argon, durchzuführen.

Zusätzlich zu den Edukten können dem Fachmann bekannte Schauminhibitoren, wie beispielsweise Decalin und Tetralin, eingesetzt werden, ohne die Umsetzung nachteilig zu beeinflussen.

Nach der Umsetzung kann das Produkt durch dem Fachmann allgemein bekannte Methoden gereinigt werden. Dazu gehören beispielsweise Umkristallisieren oder eine chromatographische Trennung. Bevorzugt lässt sich das Dicarbonsäureimid (I) durch Umkristallisieren aus einem geeigneten Lösemittel oder Lösemittelgemisch reinigen. Als Lösemittel können hierfür unter anderem Methanol, Ethanol, Dimethylformamid (DMF), Wasser und Ethylether verwendet werden. Als Gemische eignen sich Gemische aus DMF und Wasser, aus Ethylether und Methanol sowie aus Ethylether und Ethanol.

Durch die Umsetzung in Lösung treten die bekannten Probleme bei Umsetzungen in der Schmelze nicht auf. Das Produkt lässt sich leicht von möglichen Verunreinigungen wie Nebenprodukten oder Resten von den Edukten abtrennen. Das oftmals schwierige Lösen der erstarrten Schmelze entfällt. Die Reaktionsbedingungen können durch die für die Reaktionsführung in Lösung sehr gut ausgearbeiteten Verfahren leicht kontrolliert werden.

Im folgenden soll die Erfindung anhand von Beispielen näher erläutert werden, ohne darauf beschränkt zu sein.

Umsetzung von Dicarbonsäure- Anhydriden mit Harnstoff zu deren Imiden in verschiedenen Lösemitteln

### Umsetzungen von Phthalsäureanhydrid mit Harnstoff

### Beispiel 1

- 50 g (0.34 mol): Phthalsäureanhydrid wurden in
- 75 g: Diphenylether suspendiert und unter N₂ - Spülung auf 175°C erhitzt.
Nach Erreichen der Reaktionstemperatur (175°C) wurden
- 29.2 g (0.49 mol): Harnstoff eingestreut (exotherm).
Das Reaktionsgemisch wurde 30 min bei einer Innentemperatur von 170°C unter ständigem Einleiten von N₂ gerührt. Anschließend wurde auf eine Innentemperatur von ca. 90°C abgekühlt. Nach Erreichen dieser Temperatur wurden
- 300 g: Ethanol schnell zugegeben. Die entstandene Suspension wurde abfiltriert und der Filterrückstand mit Ethanol/Wasser (70/30 w/w) gewaschen.
Man erhielt Phthalimid als einen farblosen, kristallinen Feststoff mit einer Ausbeute von 68 % d. Th.

### Beispiel 2

Analog zu Beispiel 1 wurde eine Umsetzung mit Sulfolan als Lösemittel durchgeführt. Die Reaktionstemperatur betrug 180 - 185°C. Die Ausbeute betrug 66.% d. Th.

### Beispiel 3

Analog zu Beispiel 1 wurde eine Umsetzung mit N,N-Dimethylacetamid als Lösemittel durchgeführt. Die Reaktionstemperatur wurde auf 160°C begrenzt. Die Ausbeute betrug 69 % d. Th.

### Umsetzungen von Phthaloyl-Glutaminsäure-Anhydrid mit Harnstoff

### Beispiel 4

- 50 g (0.193 mol): Phthaloyl-Glutaminsäure-Anhydrid wurden in
- 75 g: Diethylenglycol-diethylether auf 180°C erhitzt.
nach Erreichen der Reaktionstemperatur wurden
- 16.5 g (0.275 mol): Harnstoff unter ständiger N₂-Spülung eingestreut (exotherm).
Anschließend wurde 1 Stunde bei Reaktionstemperatur unter anhaltender N₂- Spülung nachgerührt.
Am Ende der Reaktionszeit wurde mit Dimethylsulfoxid (DMSO) verdünnt, angekühlt und anschließend mit Ethanol versetzt.
Nach dem Abfiltrieren, Waschen und Trocknen wurden 24,9 g (49 % d.Th) Thalidomid gewonnen.

### Beispiel 5

Analog zu Beispiel 4 wurde eine Umsetzung mit Pseudocumol als Lösemittel durchgeführt. Die Reaktionstemperatur betrug 160°C. Thalidomid wurde mit 25 %iger Ausbeute isoliert.

### Beispiel 6

Analog zu Beispiel 4 wurde eine Umsetzung mit Cumol als Lösemittel durchgeführt. Die Reaktionstemperatur betrug 150°C. Thalidomid wurde mit 11 %iger Ausbeute isoliert.

### Beispiel 7

Analog zu Beispiel 4 wurde eine Umsetzung mit Mesitylen als Lösemittel durchgeführt. Die Reaktionstemperatur betrug 160°C. Thalidomid wurde mit 23%iger Ausbeute isoliert.

### Beispiel 8

Analog zu Beispiel 4 wurde eine Umsetzung mit Diethylbenzol als Lösemittel durchgeführt. Die Reaktionstemperatur betrug 170°C. Thalidomid wurde mit 39 % iger Ausbeute isoliert.

### Beispiel 9

Analog zu Beispiel 4 wurde eine Umsetzung mit 1-Ethyl-3-methyl-imidazoliumtosylat als Lösemittel durchgeführt. Die Reaktionstemperatur betrug 185°C. Thalidomid wurde mit 34 %iger Ausbeute isoliert.

### Beispiel 10

Analog zu Beispiel 4 wurde eine Umsetzung mit Decamethylcyclopentasiloxan als Lösemittel durchgeführt. Die Reaktionstemperatur betrug 180°C. Thalidomid konnte mit 20 %iger Ausbeute isoliert werden.

### Beispiel 11

Analog zu Beispiel 4 wurde eine Umsetzung mit Diphenylether als Lösemittel durchgeführt. Die Reaktionstemperatur betrug 185°C. Thalidomid konnte mit 38 %iger Ausbeute isoliert werden.

### Beispiel 12

Analog zu Beispiel 4 wurde eine Umsetzung mit Tetralin als Lösemittel durchgeführt. Die Reaktionstemperatur betrug 180°C. Thalidomid wurde mit 50 %iger Ausbeute isoliert.

### Beispiel 13

Analog zu Beispiel 4 wurde eine Umsetzung mit Decalin als Lösemittel durchgeführt. Die Reaktionstemperatur betrug 180°C. Thalidomid wurde mit 48 %iger Ausbeute isoliert.

### Beispiel 14

- 50 g (0.193 mol): Phthaloyl-Glutaminsäureanhydrid wurden in
- 75 g: NMP auf 180°C erhitzt.
nach Erreichen der Reaktionstemperatur wurden
- 16.5 g (0.275 mol): Harnstoff unter ständiger N₂-Spülung eingestreut (exotherm).
Anschliessend wurde 1 Stunde bei Reaktionstemperatur unter anhaltender N₂- Spülung nachgerührt.
Am Ende der Reaktionszeit wurde angekühlt und anschließend mit Ethanol versetzt.
Nach dem Abfiltrieren, Waschen und Trocknen wurden 19,3 g (38% d.Th) Thalidomid gewonnen.

### Beispiel 15

Analog zu Beispiel 14 wurde als Lösemittel Polyethylenglycol 400 bei 185°C eingesetzt. Thalidomid wurde mit 46 %iger Ausbeute isoliert.

### Beispiel 16

Analog zu Beispiel 14 wurde als Lösemittel Propylencarbonat bei 180°C eingesetzt. Thalidomid konnte mit 30 %iger Ausbeute isoliert werden.

### Beispiel 17

Analog zu Beispiel 14 wurde als Lösemittel Sulfolan bei 180°C eingesetzt. Thalidomid wurde mit 48 %iger Ausbeute isoliert.

### Beispiel 18

Analog zu Beispiel 14 wurde als Lösemittel N,N-Diethylanilin bei 180°C eingesetzt. Thalidomid wurde mit 49 %iger Ausbeute isoliert.

### Beispiel 19

Analog zu Beispiel 14 wurde als Lösemittel 1,3-Dimethyl-2-imidazolidinon (DMI) bei 185° eingesetzt. Thalidomid konnte mit 40 %iger Ausbeute isoliert werden.

### Beispiel 20

Analog zu Beispiel 14 wurde als Lösemittel Formamid bei 185°C eingesetzt. Thalidomid konnte mit 35 %iger Ausbeute isoliert werden.

### Beispiel 21

- 75 g: Sulfolan wurden auf 175°C erhitzt. Bei dieser Temperatur wurde ein Gemisch aus
- 50 g (0.193 mol): Phthaloyl-Glutaminsäure-Anhydrid und
- 16.5 g (0.275 mol): Harnstoff unter ständiger N₂-Spülung eingestreut. Anschliessend wurde ca. 2 Stunden bei ca. 180°C unter anhaltender N₂- Spülung nachgerührt.
Am Ende der Reaktionszeit wurde angekühlt und anschließend mit
- 285 g: Ethanol versetzt.
Nach dem Abfiltrieren, Waschen und Trocknen wurden 24,3 g (48% d.Th) Thalidomid gewonnen.

### Umsetzungen von Adipinsäure-Anhydrid mit Harnstoff

### Beispiel 22

- 20 g (0.156 mol): Adipinsäure-Anhydrid wurden in
- 30 g: Sulfolan auf eine Reaktionstemperatur von 180°C erhitzt.
Nach Erreichen der Reaktionstemperatur wurden
- 13.5 g (0.24 mol): Harnstoff eingestreut und 1h bei Reaktionstemperatur unter N₂ Spülung nachgerührt. Nach dem Abkühlen wurde die Reaktionsmischung zuerst mit 2-Propanol und dann mit Methyl-tert-butylether (MTBE) versetzt. Adipinsäure-Imid wurde mit 76 % iger Ausbeute isoliert.

### Beispiel 23

Analog zu Beispiel 23 wurde als Lösemittel Diethylenglycol-Diethylether bei 180°C eingesetzt. Adipinsäure-imid wurde mit 56 %iger Ausbeute isoliert.

### Unsetzungen von 2-Methyl-Bersteinsäure-anhydrid mit Harnstoff

### Beispiel 24

- 25 g (0.219 mol): 2-Methyl-Bersteinsäure-anhydrid wurden in
- 37.5 g: Sulfolan auf eine Reaktionstemperatur von 180°C erhitzt.
Nach Erreichen der Reaktionstemperatur wurden
- 18.95 g(0.32 mol): Harnstoff eingestreut und 1h bei Reaktionstemperatur unter N₂ Spülung nachgerührt. Nach dem Abkühlen wurde die Reaktionslösung zuerst mit 2-Propanol und anschließend mit MTBE versetzt.
Es wurden 6.5 g 2-Methyl-Bernsteinsäureimid (32 %d. Th.) gewonnen

### Beispiel 25

Analog zu Beispiel 24 wurde als Lösemittel Diethylenglycol-diethylether bei 180°C verwendet. Nach dem Abkühlen wurde zunächst mit MTBE versetzt. Aus dem entstandenen Öl wurden durch Umkristallisation aus Ethanol 2-Methyl-Bernsteinsäureimid mit 20 %iger Ausbeute gewonnen.

## Patentansprüche

1. Verfahren zur Herstellung eines Dicarbonsäureimides der allgemeinen Formel (I)
R¹-(CO)-(NR³)-(CO)-R² (I),
wobei ein Dicarbonsäureanhydrid der allgemeinen Formel (II)
R¹-(CO)-O-(CO)-R² (II)
mit Harnstoff oder einem Harnstoffderivat der Form (R³HN)-(CO)-(NR³H) in einem Lösungsmittel zu dem Dicarbonsäureimid (I) umgesetzt wird,
worin
R¹, R² und R³ unabhängig voneinander substituiertes oder unsubsituiertes, geradkettiges, verzweigtes oder cyclisches C₁-C₁₀-Alkyl, C₂-C₁₀-Alkenyl, C₂-C₁₀-Alkinyl, C₄-C₁₀-Aryl, C₄-C₁₀-Heteroaryl sein können, oder wobei R¹ und R² zur Bildung eines Ringes miteinander verbunden sein können, und/oder wobei R³ auch H sein kann.

2. Verfahren nach Anspruch 1 zur Herstellung von Dicarbonsäureimiden der allgemeinen Formel (III) 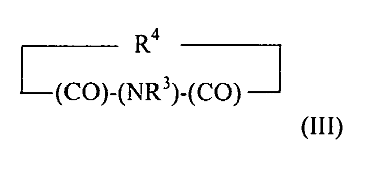 wobei
R³ wie in Anspruch 1 definiert ist, und
R⁴ ein substituiertes oder unsubsituiertes, geradkettiges, verzweigtes oder cyclisches C₁-C₁₀-Alkandiyl, C₂-C₁₀-Alkenylen, C₂-C₁₀-Alkinylen, C₄-C₁₀-Arylen, C₄-C₁₀-Heteroarylen sein kann.

3. Verfahren nach Anspruch 2 zur Herstellung von substituierten oder unsubstituierten Piperidin-2,6-dionen, wobei R⁴ ein substituiertes oder unsubsituiertes 1,3-Propandiyl ist.

4. Verfahren nach Anspruch 3 zur Herstellung von unsubstituierten oder substituierten 3-Phthalimidopiperidin-2,6-dionen, wobei R⁴ ein unsubstituiertes oder substituiertes 1-Phthalimido-1,3-propandiyl ist.

5. Verfahren nach einem oder mehreren der vorhergehenden Ansprüche, wobei das Lösungsmittel ein hochsiedendes Lösungsmittel mit einem Siedepunkt von über 150°C, bevorzugt von über 170°C, am bevorzugtesten von über 190°C ist.

6. Verfahren nach einem oder mehreren der vorhergehenden Ansprüche, wobei das Lösungsmittel ausgewählt ist aus der Gruppe, bestehend aus aprotischen Sulfonen, gesättigten Lactamen, Carbonsäureamiden, Ethern, Harnstoffen, Polyethylenglycolen, ein- oder mehrfach alkylsubstituierten Aromaten, ionischen Flüssigkeiten, Siloxanen, gesättigten oder teilweise gesättigten Carbocyclen, Kohlensäureestern, aromatischen Aminen, oder deren Mischungen.

7. Verfahren nach Anspruch 6, wobei das aprotische Sulfon Tetrahydrothiophen-1,1-dioxid (Sulfolan) ist, das gesättigte Lactam N-Methylpyrrolidon (NMP) ist, das Carbonsäureamid N,N-Dimethylacetamid (DMA) oder Formamid ist, der Ether Diphenylether ist, der Harnstoff 1,3-Dimethyl-2-imidazolidinon (DMI) ist, das Polyethylenglycol Diethylenglycol-diethylether ist, der ein- oder mehrfach alkylsubstituierte Aromat ausgewählt aus Diethylbenzol, Pseudocumol, Cumol oder Mesitylen ist, die ionische Flüssigkeit 1-Ethyl-3-methyl-imidazoliumtosylat ist, das Siloxan Decamethylcyclopentasiloxan ist, der gesättigte oder teilweise gesättigte Carbocyclus Decalin oder Tetralin ist, der Kohlensäureester Propylencarbonat ist, und/oder das aromatische Amin N,N-Diethylanilin ist, und bevorzugt als aprotisches Sulfon Tetrahydrothiophen-1,1-dioxid verwendet wird.

8. Verfahren nach einem oder mehreren der vorhergehenden Ansprüche, wobei die Temperatur bei der Umsetzung in einem Bereich von 140°C bis 220°C, bevorzugt im Bereich von 150°C bis 210°C, noch bevorzugter in einem Bereich von 160°C bis 200°C liegt.

9. Verfahren nach einem oder mehreren der vorhergehenden Ansprüche, wobei die Stoffe bei Normaldruck umgesetzt werden.

10. Verfahren nach einem oder mehreren der vorhergehenden Ansprüche, wobei zusätzlich ein Schauminhibitor eingesetzt wird.

11. Verfahren nach Anspruch 10, wobei der Schauminhibitor ausgewählt ist aus der Gruppe, bestehend aus Decalin und Tetralin.

12. Verfahren nach einem oder mehreren der vorhergehenden Ansprüche, wobei das Dicarbonsäureimid (I) in einem anschließenden Schritt durch Umkristallisieren oder mittels chromatographischer Trennmethoden gereinigt wird.

13. Verfahren nach Anspruch 12, wobei zum Umkrisallisieren des Dicarbonsäureimids (I) ein geeignetes Lösungsmittel oder Lösungsmittelgemisch, bevorzugt ein Lösungsmittel oder Lösungsmittelgemisch, ausgewählt aus der Gruppe, bestehend aus Methanol, Ethanol, einem Gemisch aus DMF und Wasser, einem Gemisch aus Ethylether und Methanol und einem Gemisch aus Ethylether und Ethanol eingesetzt wird.
